# EUROPEAN PATENT SPECIFICATION

(11) **EP 0 713 867 B1**
(45) Date of publication and mention of the grant of the patent: **16.02.2000**
(21) Application number: 95203240.7
(22) Date of filing: 24.11.1995
(51) Int. Cl.: C07C 323/52, C10M 105/72

(54) **Lubricating oil compositions**
Schmierölzusammensetzungen
Compositions d'huile lubrifiante

(30) Priority: 25.11.1994 EP 94308712
(43) Date of publication of application: 29.05.1996
(73) Proprietor: SHELL INTERNATIONALE RESEARCH MAATSCHAPPIJ B.V., 2596 HR Den Haag (NL)
(72) Inventor: Gadd, Paul George, Surbiton, Surrey KT6 4AR (GB); Gillespie, Harold Moncrieff, Chester CH4 7RQ (GB); Heywood, Frederick William, Willaston, South Wirral L64 1TW (GB); McKenna, Eugene Gerard, Brighton 3186, Victoria (AU)

(56) References cited:
- GB-A- 699 440
- US-A- 3 247 109
- US-A- 3 345 327
- US-A- 3 637 586
- US-A- 4 076 639
- US-A- 4 399 075
- PATENT ABSTRACTS OF JAPAN vol. 12 no. 501 (C-556) ,27 December 1988 & JP-A-63 210195 (KAO) 31 August 1988,
- PATENT ABSTRACTS OF JAPAN vol. 12 no. 501 (C-556) ,27 December 1988 & JP-A-63 210194 (KAO) 31 August 1988,
- CHEMICAL ABSTRACTS, vol. 78, no. 21, 28 May 1973 Columbus, Ohio, US; abstract no. 135592a, M.G. GADZHIEVA, ET AL.: 'Reaction of heptanethiol' page 326; & UCH. ZAP., AZERB. UNIV., SER KHIM. NAUK, 1972, (1), 74-76,
- BULLETIN OF THE ACADEMY OF SCIENCES OF THE USSR, DIVISION OF CHEMICAL SCIENCE, vol. 40, no. 1/2, January 1991 NEW YORK, US, pages 240-241, M.E. NIYAZYMBETOV, ET AL.: 'Electrocatalytic addition of thiols to activated olefins'
- JOURNAL OF THE CHEMICAL SOCIETY, PERKIN TRANSACTIONS II, no. 12, December 1989 LETCHWORTH, GB, pages 2121-2125, M.S.F. LIE KEN JIE, ET AL.: '1H and 13C NMR studies on the positional isomers of methyl thialaurate and methyl thiastearate'
- JOURNAL OF THE AMERICAN CHEMICAL SOCIETY, vol. 42, 1920 WASHINGTON, DC, US, pages 2385-2389, Y. UYEDA, ET AL.: 'A sulphide acid or the butyl ether of thioglycolic acid'
- JOURNAL OF ORGANIC CHEMISTRY, vol. 35, no. 3, March 1970 WASHINGTON, DC, US, pages 584-592, P.Y. JOHNSON, ET AL.: 'Photochemical reactions of gamma-keto sulphides'
- JOURNAL OF ORGANIC CHEMISTRY, vol. 24, no. 6, 14 July 1959 WASHINGTON, DC, US, pages 849-852, D.M. BURNESS: 'Decarboxylation of thietin salts'
- JOURNAL OF THE AMERICAN CHEMICAL SOCIETY, vol. 79, no. 2, 29 January 1957 WASHINGTON, DC, US, pages 362-365, N.H. KOENIG, ET AL.: 'Oragnic sulphur derivatives. I. Addition of mercaptoacetic acid to long-chain monounsaturated compounds'
- JOURNAL OF THE AMERICAN CHEMICAL SOCIETY, vol. 70, no. 7, 2 August 1948 WASHINGTON, DC, US, pages 2428-2429, J.F. MULVANEY, ET AL.: 'Esters of (carboxymethylmercapto)succinic acids'
- JOURNAL OF THE AMERICAN CHEMICAL SOCIETY, vol. 75, no. 5, 11 June 1953 WASHINGTON, DC, US, page 2765 R. FILLER, ET AL.: 'Triesters of carboxymethylmercaptosuccinic acids'
- NATURE, vol. 198, no. 4875, 6 April 1963 LONDON, GB, pages 83-84, P.H. CALDERBANK, ET AL.: 'Rupture of xanthate molecules at copper surfaces'
- JOURNAL OF APPLIED CHEMISTRY OF USSR, vol. 49, no. 9(2), September 1976 NEW YORK, US, pages 2114-2116, P.S. BELOV, ET AL.: 'Synthesis of higher esters of 7,7'-sulphinyldienanthic acid'
- CHEMICAL ABSTRACTS, vol. 59, no. 11, 25 November 1963 Columbus, Ohio, US; abstract no. 12638f, MING-CH'IEN CHIANG, ET AL.: 'Preparation of secondary alkyl esters of delta,delta'-thiodivaleric acid' & HUA HSUEH HSUEH PAO, 1962, 28(5), 347-348
- CHEMICAL ABSTRACTS, vol. 93, no. 20, 17 November 1980 Columbus, Ohio, US; abstract no. 188791f, P.S. BELOV, ET AL.: 'Synthesis and study of the antiwear action of higher esters of 5,5'-thiodivaleric acid' page 139; & KHIM. TEKHNOL. TOPL. MASEL, 1980, (7), 36-37
- CHEMICAL ABSTRACTS, vol. 83, no. 18, 3 November 1975 Columbus, Ohio, US; abstract no. 149938h, V.A. TROFIMOV, ET AL.: 'Antiwear properties of symmetrically substituted sulphides and sulphoxides' page 168; & KHIM. TEKHNOL. TOPL. MASEL, 1975, (3), 33-36
- JOURNAL OF APPLIED CHEMISTRY OF USSR, vol. 43, no. 9, September 1970 NEW YORK, US, pages 2146-2148, XP 000565473 V.I. ISAGULYANTS, ET AL.: 'Transesterification of diethyl esters of thiodicarboxylic acids with higher alcohols in the presence of KU-2/8 cation exchange resin'
- PROCEEDINGS OF THE ROYAL IRISH ACADEMY, SECTION B, vol. 51, 1947 DUBLIN, IE, pages 223-228, XP 000565504 V.C. BARRY, ET AL.: 'Antitubercular substances. IV. S-n-alkylthiomalic acids'
- CHEMISCHE TECHNIK, vol. 9, no. 3, March 1957 BERLIN, DE, pages 139-150, XP 000565829 A.N. NESMEJANOW, ET AL.: 'Die Anwendung der telomerisationsreaktion zur Synthese von omega-Aminocarbonsäuren und die Herstellung neuer Polyamidfasern auf ihrer Basis'

## Description

The present invention relates to a lubricating oil composition containing as antioxident base fluid sulphur-containing compounds, and their use as base fluids in lubricating oil compositions.

Pressures on the oxidative stability of engine lubricants are continuing to increase through a combination of longer oil drain intervals in some applications, locally higher operating temperatures, and the need for enhanced engine cleanliness to maintain low vehicle exhaust emission performance. In addition, pressures to improve automotive vehicle fuel economy have led to an increased requirement for low viscosity engine oils. Furthermore, there is a need for base fluids that combine low viscosity with low volatility. A high viscosity index in the base fluid can also contribute to meeting viscosity and volatility targets.

Improved oxidation resistance can be achieved in lubricant formulation by use of antioxidant additives, by use of intrinsically more stable lubricant base fluids, or by a combination of these approaches. The compounds of the present invention impart excellent oxidation resistance to lubricating oil compositions containing these compounds as base fluids through antioxidant functionality incorporated in the compound molecule, whilst also maintaining the combination of other physical fluid properties (viscosity, viscosity index, pour point and volatility) required for blending lubricating oils. Some antioxidant additives in current use suffer from practical disadvantages, for lubricating oil applications, of being solids or having relatively high volatility; such disadvantages are avoided by the compounds of the present invention.

Magne et al., J. Am. Oil Chem. Soc. 1975, 52, pages 494-497 describes a number of fatty acid esters as lubricants and lubricant additives, being particularly concerned with anti-wear and extreme pressure performance. Sample 36 in that document is 2-(2-ethoxyethoxy)-ethyl-9(10)-(carbobutoxymethylthio)-stearate. However, as discussed below, it has been found that said compound displays unacceptable incompatibility with elastomer seals.

GB-A-863339 discloses extreme-pressure lubricants which comprise a minor proportion of an oil-soluble aliphatic compound having as substituents at least one oxygen-containing group Y and from 1 to 4 groups of the formula -S-(CH₂)ₙ-X, wherein Y is an alcohol, ether, carboxylic acid or ester group, n is 1 to 4, and X is the group -OR or -COOR, where R is a hydrogen atom or a C₁-C₄ alkyl group, at least one of the -S-(CH₂)ₙ-X groups being separated from a group Y by a chain of at least 7 carbon atoms. Of the Examples in GB-A-863339, only Example II discloses an ester, which was tested, in Composition E, only for its extreme-pressure properties (Table II).

US-A-3247109 relates to a lubricating oil composition comprising a major proportion of dialkyl esters of alkoxysuccinic acids and alkylthiosuccinic acids.

US-A-4076639 discloses lubricant media containing a minor antiwear improving amount, preferably from about 0.1 to about 2% by weight, of certain alkylbetathiopropionic acid esters. The amount used in the examples is between 0.44 and 1.21% wt.

GB 699440 relates to esters of decarboxylic acids containing one or more atoms of sulfur in thioether linkages. The esters can be used as such as lubricating oil or may be blended with mineral lubricating oils.

Commercially available antioxidants which contain one sulphide link and two carboxylic ester groups include thiodipropionates of the formula S(CH₂CH₂CO₂R)₂, for example 'Irganox PS800' by Ciba-Geigy, where R is n-dodecyl, and 'DTDTDP' by Akcros, where R is n-tridecyl. However, the first of those compounds is a solid and is therefore entirely unsuitable as a lubricant base fluid. The second of those compounds, although being a liquid, is marketed as a polymer stabiliser, that is, for an application quite unrelated to that to which the present invention is directed.

In accordance with the present invention there is provided a lubricating oil composition comprising as antioxident base fluid a compound of the formula 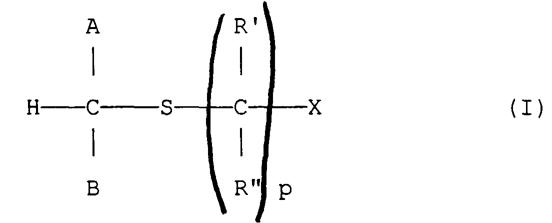 wherein
A is a hydrogen atom or an alkyl group;
B is an alkyl group; or a group of the formula CO₂-W, where W is an alkyl group; or a group of the formula Y-CO₂-Z, where Y is an alkylene group and Z is an alkyl group; or a group of the formula CH₂CH(CO₂D)CH₂CO₂E, where D and E are each independently alkyl groups;
R' and R" are each independently a hydrogen atom, an alkyl group or a group CH₂CO₂F, where F is an alkyl group;
p is 0 to 40;
X is a group CO₂G where G is a C₁₋₃₀ alkyl group, or a group (CH₂)ₙCO₂J where J is a C₁₋₁₀ alkyl group and n is 1 to 12;
said compound having a molecular weight of at least 440; with the exclusion of
(a) compounds of the formula S(CH₂CH₂CO₂R)₂ or S(CH₂CO₂R)₂, wherein R is an alkyl group,
(b) compounds of the formula 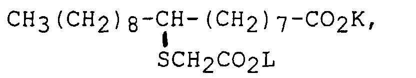 wherein K and L are each an octyl group or wherein K is a butyl group and L is an ethyl group,
(c) a compound of the formula 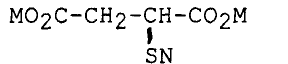 wherein M is a decyl group and N is a dodecyl group, and
(d) alkyl beta-thio propionic acid ester selected from the group consisting of R₁SCH₂CH₂CO₂R₂ wherein R₁ and R₂ are alkyl groups containing from 1 to 30 carbon atoms, and
wherein the said compound is present in the amount of 5 to 25% by weight of the total base fluid.

In the compounds of formula I above, preferably:
A is a hydrogen atom or a C₁₋₃₀ alkyl group, more preferably a hydrogen atom or a C₁₋₁₆ alkyl group;
B is a C₁₋₃₀ alkyl group, more preferably a C₁₋₁₆ alkyl group; or a group CO₂-W where W is a C₁₋₃₀ alkyl group, more preferably a C₃₋₁₂ alkyl group; or a group Y-CO₂-Z where Y is an alkylene group of carbon number C₁₋₃₀, more preferably 1 to 15, and Z is a C₁₋₃₀ alkyl group, more preferably a C₄₋₁₂ alkyl group; or a group CH₂CH(CO₂D)CH₂CO₂E where D and E are each independently C₃₋₁₅ alkyl groups, more preferably C₇₋₉ alkyl groups;
R' and R" are each independently a hydrogen atom; or a C₁₋₁₀ alkyl group, more preferably a butyl group; or a group CH₂CO₂F where F is a C₃₋₁₅ alkyl group, more preferably a C₇₋₉ alkyl group;
p is 1 or 2; and
X is a group CO₂G where G is a C₁₋₁₂ alkyl group; or a group (CH₂)ₙCO₂J where J is a hexyl group, and n is 7 to 8.

In the lubricating oil composition according to the present invention, the said compound is present in the amount of 5 to 25%, by weight of the total base fluid.

Furthermore, in the lubricating oil composition according to the present invention, the balance of the total base fluid is preferably a base oil of mineral or synthetic origin.

In accordance with the present invention there is still further provided use of a compound of the formula 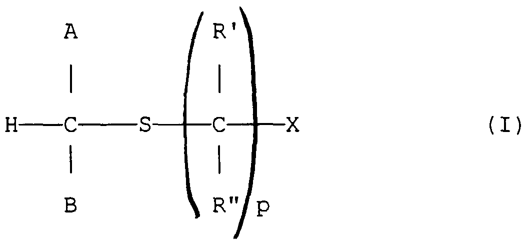 wherein
A is a hydrogen atom or an alkyl group;
B is an alkyl group; or a group of the formula CO₂-W, where W is an alkyl group; or a group of the formula Y-CO₂-Z, where Y is an alkylene group and Z is an alkyl group; or a group of the formula CH₂CH(CO₂D)CH₂CO₂E, where D and E are each independently alkyl groups;
R' and R" are each independently a hydrogen atom, an alkyl group or a group CH₂CO₂F, where F is an alkyl group;
p is 0 to 40;
X is a group CO₂G where G is a C₁₋₃₀ alkyl group, or a group (CH₂)ₙCO₂J where J is a C₁₋₁₀ alkyl group and n is 1 to 12;
with the exclusion of compounds of the formula S(CH₂CH₂CO₂R)₂ or S(CH₂CO₂R)₂, wherein R is an alkyl group;
as an antioxidant base fluid in a lubricating oil composition wherein the said compound is present in the amount of 5 to 25% by weight of the total base fluid.

Suitable olefinically unsaturated starting materials that may be used in the preparation of the sulphur-containing compounds include:
(a) alpha olefins; internal olefins obtained by partial isomerisation of alpha olefins, where unsaturation is principally but not exclusively in the β C=C bond; internal olefins obtained by complete isomerisation of alpha olefins, where C=C bonds are distributed randomly over all possible positions in the hydrocarbon chain; internal olefins where the C=C bond is in a particular position in the hydrocarbon chain, for example centrally; olefins obtained by oligomerisation of propene or butenes; olefins obtained by dimerisation or higher oligomerisation of alpha or internal olefins higher than C₄, for example C₈, C₁₀ or C₁₂ olefins. Olefins obtained by cracking of higher molecular weight materials, for example waxes. The carbon number of said olefins is C₈₋₁₀₀₀, preferably C₁₀₋₁₀₀, most preferably C₁₆₋₄₀. The olefin may be linear, branched or cyclic and may be further substituted with other groups such as aryl groups;
(b) plant oils, for example triglycerides, in which at least 60% by weight of constituent fatty acids have at least one C=C bond, for example soya oil, sunflower oil, corn oil, high erucic rapeseed oil, low erucic rapeseed oil, olive oil, tall oil, groundnut oil, castor oil, vernonia oil; derivatives of such plant oils, for example their constituent fatty acids such as oleic acid, linoleic acid, linolenic acid, erucic acid, ricinoleic acid, docosenoic acid; or esters of such fatty acids, for example methyl oleate, methyl linoleate, glycerol trioleate;
(c) organic acids with at least one C=C bond, preferably alkenoic acids R-C=C-Y-CO₂H where R is alkyl, aryl, or acyl of carbon number C₁₋₁₀₀ and Y is an alkylene group of carbon number C₁₋₃₀.

Alternatively, compounds of the claimed structure may be prepared by reaction of an olefinically unsaturated acid anhydride, for example maleic anhydride.

The compounds of the present invention may be prepared by known methods, for example by electrophilic, nucleophilic or free-radical addition (either with or without the use of a free-radical initiator) of a suitable sulphur-containing compound, for example hydrogen sulphide, a thiol, a thiophenol, or a thiol acid, for example mercaptoacetic acid, with one of the olefinically unsaturated materials mentioned above, for example an olefin or an olefinically unsaturated acid, for example oleic acid. Suitable free-radical initiators include organic peroxides, for example lauroyl peroxide. The resulting intermediate may contain one or more carboxylic acid groups which may be esterified by reaction with an alcohol or mixture of alcohols catalysed by for example an acid, such as sulphuric acid, 4-toluenesulphonic acid, methanesulphonic acid, or by a metal alkoxide, for example titanium isopropoxide, or by acid sites in an ion exchange resin, or may be esterified by uncatalysed reaction. Where the intermediate already contains ester groups, for example, methyl esters, these ester groups may be transesterified by acid catalysed reaction with another alcohol or mixture of alcohols using acid catalysts as listed above. Suitable alcohols include linear primary alcohols, for example linear primary C₃₋₂₀ alcohols, preferably linear primary C₅₋₁₀ alcohols; or branched alcohols in the same carbon number range, for example having one or more methyl or ethyl substituents.

Suitable additives with which the compounds of the present invention may be used for engine oil application may include one or more extreme pressure/antiwear agents, for example zinc salts such as zinc dialkyl or diaryl phosphorodithioates (at a concentration giving from 0 to 0.15% w/w phosphorus in the lubricant); one or more overbased metal-containing detergents such as calcium or magnesium alkyl salicylates or alkylarylsulphonates (present at total detergent metal concentration in the lubricant from 0 to 2% w/w); one or more ashless dispersant additives such as reaction products of polyisobutenyl succinic anhydride and an amine or ester (present at total dispersant active matter concentration in the lubricant from 0 to 10% w/w); optionally one or more primary antioxidants, for example hindered phenols or amines (present at concentrations in the lubricant from 0 to 5% w/w). Other supplementary additives, for example antirust or friction-modifying additives may optionally be present. Viscosity Index improving polymers may optionally be present at polymer concentrations from 0 to 10% w/w. Pour point depressing additives may optionally be present at concentrations from 0 to 5% w/w.

The balance of base oil in the lubricant may be a base oil of mineral or synthetic origin. Base oils of mineral origin may be mineral oils, for example produced by solvent refining or hydroprocessing. Base oils of synthetic origin may typically be mixtures of C₁₀₋₅₀ hydrocarbon polymers, for example liquid polymers of alpha-olefins. They may also be conventional esters, for example polyol esters. The base oil may also be a mixture of these oils. Preferably the base oil is that of mineral origin sold by the Royal Dutch/Shell Group of Companies under the designations "HVI" or, most preferably, the synthetic hydrocarbon base oils sold by the Royal Dutch/Shell Group of Companies under the designation "XHVI" (trade mark).

The following Examples 1 to 15 illustrate the preparation of compounds of the present invention:

### EXAMPLE 1

### Preparation of hexyl 9(10)-(hexyloxycarbonylmethylthio)octadecanoate

70% oleic acid (12 kg, 42.49 moles), freshly distilled mercaptoacetic acid (5.9 kg, 64.05 moles) and lauroyl peroxide (0.1 kg) were stirred and heated to 60°C under a nitrogen atmosphere. Further portions of peroxide (each 0.1 kg) were added at 3 hour intervals to a total reaction time of 45 hours. The mixture was heated at 90°C for a further two hours, before being cooled to ambient temperature and diluted with toluene (17 l). The toluene solution was washed with water (5 x 8 l), dried by azeotropic distillation, and n-hexanol (14 l) and methanesulphonic acid (0.32 kg) added. The reaction was warmed to reflux until the vapour temperature rose to 110°C, and distillation then continued for a further 4 hours. After cooling to ambient temperature, the solution was washed with water (4 l) and aqueous sodium bicarbonate solution (4 l), dried (Na₂SO₄) and filtered. The filtrate was stirred successively overnight with pre-activated 'Amberlyst A-26' ion exchange resin (2 x 1 kg, 1 x 2 kg), filtered, and the solvent evaporated. Wiped film evaporation at 140°C/1 mbar and a pass rate of ca. 750 ml/hour gave 22.5 kg of residue, which was re-distilled at 185°C/1 mbar, pass rate ca. 750 ml/hour, to give hexyl 9(10)-(hexyloxycarbonylmethylthio)octadecanoate (20.2 kg) as an orange/brown oil.

### Activation of the ion-exchange resin:

'Amberlyst A-26' ion-exchange resin (2 kg) was added to a solution of concentrated hydrochloric acid (300 ml) in methanol (2.7 l) and the mixture stirred for 15 minutes. The resin was poured into a column and washed with deionised water until the washings were neutral (approx. 20 l required). After elution with 2M aqueous sodium hydroxide solution (3 l) the resin was again washed with deionised water until the washings were neutral (approx. 12 l required). The column was finally washed with methanol until the eluant showed <1% water by Karl-Fischer analysis (approx. 10 l required).

### EXAMPLE 2

### Preparation of dodecyl 9(10)-(dodecyloxycarbonylmethylthio)octadecanoate

90% oleic acid (400 g, 1.42 moles) and freshly distilled mercaptoacetic acid (196.2 g, 2.13 moles) were stirred and heated to 60°C under a nitrogen atmosphere. Lauroyl peroxide (3 g) was added and heating continued. Further portions of lauroyl peroxide (each 3 g) were added at 3 hour intervals to a total reaction time of 30 hours. The mixture was cooled, diluted with diethyl ether (500 ml), washed with water (2 x 75 ml) and dried (MgSO₄). Evaporation of the solvent gave intermediate C (606 g).

Intermediate C (300 g, 0.8 moles), n-dodecanol (447.2 g, 2.4 moles) and methanesulphonic acid (7.5 g) were dissolved in toluene and heated to reflux under a 30 plate fractionating column. Distillation was continued for approximately 2 hours after the vapour temperature reached 110°C. The solution was cooled, washed with water (2 x 250 ml) and saturated aqueous sodium bicarbonate solution (250 ml) and dried (MgSO₄). The filtered solution was stirred succesively overnight with activated 'Amberlyst A -26' resin (2 x 30 g, 2 x 50 g), filtered and the solvent evaporated. Removal of volatile impurities by wiped film distillation at 185°C/0.8 mbar gave dodecyl 9(10)-(dodecyloxycarbonylmethylthio)octadecanoate (444 g) as an oil.

### EXAMPLE 3

### Preparation of hexyl 11-(hexyloxycarbonylmethylthio)undecanoate

10-undecenoic acid (180 g, 0.98 moles) and freshly distilled mercaptoacetic acid (135 g, 1.47 moles) were stirred together, the temperature rising to 100°C. The mixture was heated to 110°C for 2 hours, and allowed to cool to 60°C. Carbon tetrachloride (300 ml) was added and the solution allowed to crystallise. The resulting white solid was filtered and washed with carbon tetrachloride and hexane, and dried in vacuo at 40°C to give 11-(carboxymethylthio)undecanoic acid (279.9 g) (Compound E).

A solution of Compound E (276 g, 1 mole), n-hexanol (276 g, 2.7 moles) and methanesulphonic acid (5.5 g) in toluene (2 l) was warmed to reflux under a 30 plate fractionating column. Distillation was continued for approximately 3 hours after the vapour temperature reached 110°C. The solution was cooled, washed with water (2 x 75 ml) and saturated aqueous sodium bicarbonate solution and dried (MgSO₄). The filtered solution was stirred successively overnight with 'Amberlyst A-26' resin (2 x 100 g, 2 x 25 g), filtered and subjected to wiped film distillation at 140°C/0.1 mbar to give 298.1 l of crude product as a yellow oil. Redistillation of 223 g of the material at 150°C/0.5 mbar gave 133.7 g of hexyl 11-(hexyloxycarbonylmethylthio)undecanoate.

### EXAMPLE 4

### Preparation of hexyl 13(14)-(hexyloxycarbonylmethylthio)docosanoate

Docosanoic acid (50 g, 0.15 moles) and freshly distilled mercaptoacetic acid (20.7 g, 0.23 moles) were warmed to 60°C under a nitrogen atmosphere. Lauroyl peroxide (0.4 g) was added and heating continued. Further portions of lauroyl peroxide (each 0.4 g) were added at 3 hour intervals to a total reaction time of 55 hours. The mixture was cooled, diluted with diethyl ether (150 ml), washed with water (2 x 25 ml) and dried (MgSO₄). Evaporation of the solvent gave intermediate F (72.0 g) as an oil. The preparation of intermediate was carried out twice.

Intermediate F (150 g, 0.35 moles), n-hexanol (96.1 g, 0.94 moles) and methanesulphonic acid (2.5 g) were dissolved in toluene (1 l) and warmed to reflux under a 30 plate fractionating column. Distillation was continued for approximately 2 hours after the vapour temperature reached 110°C. The solution was cooled, washed with water (2 x 100 l) and saturated aqueous sodium bicarbonate solution (100 ml) and dried (MgSO₄). The filtered solution was stirred successively overnight with activated 'Amberlyst A-26' resin (2 x 100 g), filtered and the solvent evaporated. Removal of volatile impurities by wiped film distillation at 180°C/0.1 mbar gave hexyl 13(14)-(hexyloxycarbonylmethylthio)docosanoate (134.7 g) as an oil.

### EXAMPLE 5

### Preparation of butyl 9(10)-(butoxycarbonylmethylthio)octadecanoate

70% oleic acid (2.4 kg, 8.5 moles), freshly distilled mercaptoacetic acid (1.17 kg, 12.75 moles) and lauroyl peroxide (24 g) were stirred and heated to 60°C under a nitrogen atmosphere. Further portions of peroxide (each 24 g) were added at 3 hour intervals to a total reaction time of 45 hours. The mixture was heated at 90°C for a further 2 hours, before being cooled to ambient temperature and diluted with toluene (11 l). The toluene solution was washed twice with water and dried (MgSO₄) to give a solution of intermediate G, which was not isolated. The solution was divided into two equal portions.

One portion of solution of intermediate G, n-butanol (1.66 kg, 22.4 moles) and methanesulphonic acid (34 g) in toluene (6 l) was warmed to reflux under a 30 plate fractionating column. Distillation was continued for approximately 2 hours after the vapour temperature reached 110°C. The solution was cooled to ambient temperature, washed with water (500 ml) and saturated aqueous sodium bicarbonate solution (200 ml) dried (MgSO₄) and filtered. The filtered solution was stirred successively overnight with activated 'Amberlyst A-26' resin (3 x 300 g), filtered and the solvent evaporated. Wiped film distillation at 160°C/1.4 mbar gave 1.71 kg of residue. Redistillation at 180°C/1.1 mbar gave butyl 9(10)-(butoxycarbonylmethylthio)octadecanoate (1.54 kg) as an orange-brown oil.

### EXAMPLE 6

### Preparation of alkyl 9(10)-(alkoxycarbonylmethylthio)octadecanoate (alkyl = mixed heptyl, octyl, nonyl; alkoxy = mixed heptyloxy, octyloxy, nonyloxy)

One portion of solution of intermediate G from Example 5, a mixture of C₇-C₉ linear primary alcohols (Shell 'Linevol 79' (trade mark)) (1.62 kg, approximately 12.7 moles) and methanesulphonic acid (34 g) in toluene (5 l) was warmed to reflux under a 30 plate fractionating column. Distillation was continued for approximately 2 hours after the vapour temperature reached 110°C. The solution was cooled to ambient temperature, washed with water (500 ml), dried (MgSO₄) and filtered. The filtered solution was stirred successively overnight with activated 'Amberlyst A-26' resin (3 x 300 g), filtered and the solvent evaporated. Wiped film distillation at 160°C/1.3 mbar gave 2.45 kg of residue. Redistillation at 180°C/1.0 mbar gave alkyl 9(10)-(alkoxycarbonylmethylthio)octadecanoate (2.22 kg) as an orange-brown oil (alkyl = heptyl, octyl, nonyl; alkoxy = heptyloxy, octyloxy, nonyloxy).

### EXAMPLE 7

### Preparation of mixed (1-9)-(alkoxycarbonylmethylthio)octadecane (alkoxy = heptyloxy, octyloxy, nonyloxy)

A fully isomerised mixture of C₁₈ olefins (161.6 g, 0.64 moles), mercaptoacetic acid (88.4 g, 0.96 moles) and lauroyl peroxide (1 g) were heated to 62°C for 6 hours, and then with addition of a further 0.5 g of lauroyl peroxide for a further 12 hours. The mixture was cooled, diluted with pentane (100 ml), washed with water and dried (Na₂SO₄). After evaporation of solvent, the intermediate was mixed with 2:1 molar excess of mixed linear primary C₇, C₈ and C₉ alcohols (Shell 'Linevol 79' (trade mark)) and 0.15 ml titanyl isopropoxide. The mixture was heated to 200°C for 4 hours, then excess alcohol was removed by distillation under vacuum. The product was dissolved in hexane and passed through silica gel (20-45 µm) to remove catalyst residues, and hexane evaporated under vacuum to give 225 g of product.

### EXAMPLE 8

### Preparation of mixed 9-(alkoxycarbonylmethylthio)octadecane (alkoxy = heptyloxy, octyloxy, nonyloxy)

9-octadecene (162 g, 0.64 moles, mercaptoacetic acid(82.9 g, 0.90 moles) and lauroyl peroxide (1 g) were heated to 80°C, with further additions of 1 g lauroyl peroxide at 3 and 8 hours, for a total reaction time of 26 hours. The mixture was cooled, an equal volume of toluene added, and washed 6 times with water. The intermediate acid was esterified with a 20% molar excess of mixed linear primary C₇, C₈ and C₉ alcohols (Shell 'Linevol 79' (trade mark)) catalysed by para-toluene sulphonic acid (1% w/w on alcohol). After 4 hours reaction, the mixture was washed with water until the pH of the aqueous phase was neutral, and solvent and unreacted alcohol and olefin were removed by vacuum distillation, to give the mixed 9-(alkoxycarbonylmethylthio)octadecane.

### EXAMPLE 9

### Preparation of mixed 11-(alkoxycarbonylmethylthio)docosane (alkoxy = heptyloxy, octyloxy, nonyloxy)

11-docosene (308.6 g, 1.0 moles) was reacted with mercaptoacetic acid (138.2 g, 1.5 moles) and the intermediate worked up and reacted with mixed linear primary C₇, C₈ and C₉ alcohols (Shell 'Linevol 79' (trade mark)) under the conditions described in Example 8. Residual olefin and alcohols were removed by wiped film evaporation at 180°C/0.2 mbar, to give the mixed 11-(alkoxycarbonylmethylthio)docosane.

### EXAMPLE 10

### Preparation of mixed (1-9)-(alkoxycarbonylmethylthio)heptadecane, (1-9)-(alkoxycarbonylmethylthio)octadecane, and (1-10)-(alkoxycarbonylmethylthio)nonadecane (alkoxy = heptyloxy, octyloxy, nonyloxy): substituents predominantly in the 2-position

A mixture of C₁₇, C₁₈ and C₁₉ internal olefins (500 g, 2.0 moles) was reacted with mercaptoacetic acid (276 g, 3.0 moles) at 80°C for 24 hours, using staged addition of lauroyl peroxide (3.0 g) as initiator. The product was diluted with toluene, washed with water, esterified with mixed linear primary C₇-C₉ alcohols (Shell 'Linevol 79' (trade mark)) using a 20% molar excess of alcohol and p-toluenesulphonic acid (PTSA) as catalyst (molar ratio PTSA:alcohol 1:100), washed and distilled in a similar manner to Example 13.

### EXAMPLE 11

### Preparation of mixed alkyl 3-alkoxycarbonyl-4(5)-(alkoxycarbonylmethylthio)nonanoate (alkyl = heptyl, octyl and nonyl; alkoxy = heptyloxy, octyloxy and nonyloxy)

This product was obtained in a similar manner to Example 12, using hexenyl succinic anhydride instead of maleic anhydride. The intermediate diester was not separated. After free radical addition of mercaptoacetic acid, the added acid group was esterified with further Shell 'Linevol 79' (trade mark).

### EXAMPLE 12

### Preparation of mixed dialkyl 2-(alkoxycarbonylmethylthio)succinate (alkyl = heptyl, octyl and nonyl; alkoxy = heptyloxy, octyloxy and nonyloxy)

Maleic anhydride (0.5 mole) was reacted with 1.6 moles of mixed linear primary C₇₋₉ alcohols (Shell 'Linevol 79' (trade mark)) until the calculated quantity of water had been removed by azeotrope. Excess alcohol was removed by distillation. The resulting diester was reacted with 50% molar excess of mercaptoacetic acid. The majority of the reaction was complete within 4 hours, but reaction was continued for 24 hours total with stepwise addition of a total of 3.0 g lauroyl peroxide. The mixture was purified by elution through a silica gel column using hexane followed by dichloromethane then finally with dimethylketone. Dimethylketone was removed by evaporation from its eluate, and the resulting residue was esterified with Shell 'Linevol 79' (trade mark) to give the product.

### EXAMPLE 13

### Preparation of mixed 11(12)-(2-alkoxycarbonylethylthio)docosane (alkoxy = heptyloxy, octyloxy and nonyloxy)

11-docosene (388 g, 1.26 moles) was reacted with a 50% molar excess of 3-mercaptopropionic acid at 80°C for 26 hours using stepwise addition of lauroyl peroxide as free radical initiator. A total of 3.0 g of lauroyl peroxide was added. The resulting intermediate was diluted with an equal volume of toluene and washed 6 times with water. The intermediate was esterified with a 20% molar excess of mixed linear primary C₇₋₉ alcohols (Shell 'Linevol 79' (trade mark)) catalysed by p-toluenesulphonic acid (1% w/w on alcohol). Reaction was complete after 4 hours. The product was washed with a slight excess of aqueous sodium hydroxide, and then with repeated water wash until the pH of the aqueous phase was neutral. Toluene, excess alcohol and unreacted olefin were removed by vacuum distillation.

### EXAMPLE 14

### Preparation of hexyl 9(10)-(2-hexyloxycarbonylethylthio)octadecanoate

Oleic acid (28.3 g, 0.10 mole) and 3-mercaptopropionic acid (16.0 g, 0.15 mole) were reacted at 60°C under nitrogen with staged addition of 3.6 g of lauroyl peroxide over 32 hours. The mixture was heated for a further 16 hours at 60°C, followed by a further 3 hours at 95°C, then cooled, diluted with diethyl ether (100 ml), washed with water, dried (MgSO₄) and the solvent removed to yield 9(10)-(carboxyethylthio)octadecanoic acid as an orange oil. Hexanol (31.4 g, 0.31 mole), methanesulphonic acid (0.84 g, 9 mmol) and toluene (150 ml) were added and the mixture was refluxed with removal of water. The mixture was cooled, washed with water, and treated with calcium hydroxide (8.5 g) and water (1 ml), then dried with MgSO₄. Solvent was removed by rotary evaporator to yield an oil (91.5 g). This was re-treated with calcium hydroxide and water, filtered, treated with anhydrous sodium carbonate and water, and filtered. The product was subjected to vacuum distillation by rotary evaporator, then wiped film evaporator, to yield 36.8 g of hexyl 9(10)-(2-hexyloxycarbonylethylthio)octadecanoate.

### EXAMPLE 15

### Preparation of hexyl-9(10)-(octylthio)octadecanoate

This was prepared analogously to Example 14, using oleic acid (28.4 g, 0.10 mole), octanethiol (23.5 g, 0.16 mole) and lauroyl peroxide (3.6 g). After esterification, treatment with calcium hydroxide (twice) and sodium carbonate, followed by wiped film evaporation gave 30.0 g of hexyl-9(10)-(octylthio)octadecanoate.

Table 1 shows the physical properties of compounds of the present invention:

**TABLE 1**

| Ex. No. | Kinematic viscosity at 100°C, mm²/s | Viscosity Index | Pour point °C | Volatility weight % loss (NOACK)* | Volatility weight % loss (TGA)** |
|---|---|---|---|---|---|
| 1 | 4.81 | 167 | -48 | 3.0 | 3.5 |
| 2 | 7.28 | 183 | -18 | | 2.0 |
| 3 | 3.35 | 174 | (a) | | 17.9 |
| 4 | 5.94 | 180 | -39 | | 1.2 |
| 5 | 4.25 | 159 | -60 | | 5.0 |
| 6 | 5.16 | 174 | -24 | | 6.0 |
| 7 | 3.40 | 148 | -31 | 7.5 | |
| 8 | 3.14 | 135 | -30 | 8.3 | |
| 9 | 4.09 | 154 | -9 | 2.8 | |
| 10 | 3.81 | 159 | -18 | | 19.1 |
| 11 | 6.63 | 141 | -57 | | 5.2 |
| 12 | 4.28 | 123 | <-60 | | 10.0 |
| 13 | 4.35 | 159 | -15 | 3.3 | |
| 14 | 4.93 | 172 | -30 | | 9.8 |
| 15 | 4.50 | 172 | | | 15.6 |

| | | | | | |
|---|---|---|---|---|---|
| (a) anomalous behaviour | | | | | |
| * The Noack method (Test Method CEC-L-40-T-87) measures the evaporative loss from an oil held at 250°C | | | | | |
| ** TGA is Test Method IP 393/91. Weight loss up to 262°C is determined from the record of mass loss as a function of temperature in a suitable thermogravimetry instrument run at a constant heating rate of 10°C/min from 40°C to 550°C under flowing nitrogen. This measurement has been shown (based on a limited correlation exercise) to provide an indication of the result to be expected by the Noack method. | | | | | |

The compounds have high viscosity indices combined with low pour points, and the viscosities of the products are in a suitable range for lubricant application and particularly for engine lubricant application.

The oxidational stability of oils was evaluated by differential scanning calorimetry (DSC). DSC measures the differential heat flow (sample vs reference) when a sample of oil in a small pan, and a reference (empty pan) are heated under an oxygen or air atmosphere in a closed cell. Suitable apparatus is commercially available from a number of suppliers. Since oxidation of oils is exothermic, DSC may be used to monitor the oxidation rate of oils. Typically a 2 mg sample of oil is used in an open aluminium pan. Experiments of two types were performed:
(a) in temperature programmed (dynamic) experiments the test temperature was ramped linearly from 40°C to 400°C at 20°C/min. The cell atmosphere was 2 bar oxygen, and the gas flow rate 60 normal ml/min. The onset of rapid oxidation is marked by a rapidly increasing exotherm. The extrapolated onset temperature is defined by the intersection of the tangent at the steepest part of the increasing exotherm and the baseline extrapolated from lower temperatures, on a plot of power vs temperature.
(b) in isothermal experiments, the temperature is held constant at a selected temperature, e.g. 220°C. The cell atmosphere was 30 bar oxygen, and the gas flow rate 60 normal ml/min. If the oil contains primary antioxidant, there is an induction period (a period in which the oxidation rate is being controlled by antioxidant so that little heat output is observed) before rapid oxidation marked by a rapidly increasing exotherm occurs. The induction period is defined by the intersection of the tangent at the steepest part of the increasing exotherm and the baseline extrapolated from earlier time, on a plot of power vs time.

Table 2 sets outs the extrapolated onset temperatures of compounds of the present invention and of a comparative polyol ester (Comparative A) and a comparative synthetic hydrocarbon (Comparative B):

**TABLE 2**

| Example No. | Extrapolated onset temperature °C |
|---|---|
| 1 | 254 |
| 2 | 281 |
| 3 | 259 |
| 4 | 271 |
| 5 | 292 |
| 6 | 284 |
| 7 | 272 |
| 8 | 287 |
| 9 | 274 |
| 10 | 285 |
| 11 | 290 |
| 12 | 253 |
| 13 | 288 |
| 14 | 283 |
| 15 | 252 |
| Comparative A | 217 |
| Comparative B | 209 |

The compounds of the present invention show substantially higher extrapolated onset temperatures than the polyol ester or synthetic hydrocarbon fluids.

Table 3 sets out induction periods measured by isothermal DSC at 220°C of lubricant compositions (Compositions B to H) containing compounds of the present invention and also a hydrocarbon base oil and an additive package (Package A) suitable for use in engine lubricants, containing zinc dithiophosphate, overbased metal detergent, ashless dispersant and a supplementary primary antioxidant. For purposes of comparison, Table 3 also includes a result for polyol ester Comparative A, which is typical of those in current use in lubricating oils, and for Comparative C (mixed dialkyl 2,2'-thiodiethanoate S(CH₂CO₂R)₂; R = mixture of branched C₁₄₋₁₈ alkyl):

**TABLE 3**

| Composition | Compound contained in composition | %w of compound in composition | Induction period, min |
|---|---|---|---|
| A* | None | 0 | 8 |
| B1 | Example 1 | 5 | 19 |
| B2 | Example 1 | 10 | 27 |
| B3 | Example 1 | 20 | 22 |
| B4* | Example 1 | 50 | 17 |
| C1 | Example 7 | 5 | 24 |
| C2 | Example 7 | 10 | 30 |
| C3 | Example 7 | 20 | 26 |
| C4* | Example 7 | 50 | 17 |
| D1 | Example 11 | 5 | 16 |
| D2 | Example 11 | 10 | 26 |
| D3 | Example 11 | 20 | 28 |
| D4* | Example 11 | 50 | 24 |
| E1 | Example 12 | 5 | 15 |
| E2 | Example 12 | 10 | 24 |
| E3 | Example 12 | 20 | 25 |
| E4* | Example 12 | 50 | 19 |
| F1 | Example 13 | 5 | 13 |
| F2 | Example 13 | 10 | 16 |
| F3 | Example 13 | 20 | 15 |
| F4* | Example 13 | 50 | 10 |
| G1 | Example 14 | 5 | 15 |
| G2 | Example 14 | 10 | 16 |
| G3 | Example 14 | 20 | 12 |
| G4* | Example 14 | 50 | 7 |
| H1 | Example 15 | 5 | 13 |
| H2 | Example 15 | 10 | 14 |
| H3 | Example 15 | 20 | 11 |
| H4* | Example 15 | 50 | 5 |
| J1* | Comparative A | 5 | 7 |
| J2* | Comparative A | 10 | 7 |
| J3* | Comparative A | 20 | 10 |
| J4* | Comparative A | 50 | 12 |
| K1* | Comparative C | 5 | 7 |
| K2* | Comparative C | 10 | 9 |
| K3* | Comparative C | 20 | 12 |
| K4* | Comparative C | 50 | 20 |

| | | | |
|---|---|---|---|
| * : Comparative | | | |

Polyol esters are known to have increased oxidational stability compared with hydrocarbon base fluids. However, when used in blends with hydrocarbon base fluids, little or no improvement in the oxidational stability of the blend was observed at lower concentrations of the ester, for example <50% by weight of the blend. Composition J incorporated Comparative A at concentrations from 5-50% by weight. Unlike Comparative A, the compounds of the present invention give a substantial benefit in oxidational stability in the blend, in oxidation tests such as DSC, at low concentration of fluid in the blend, for example 5-20% by weight.

By contrast, the compound of Comparative C, which has no hydrogen atoms in positions β to the sulphur atom, gave little benefit in this concentration range when blended as Composition K.

The fluid of Example 1 was blended at 17% by weight in a lubricant composition (Composition M), containing additive Package A, a pour point depressant and a Viscosity Index improving polymer. The balance of the blend to 100% consisted of a hydrocarbon base fluid. Table 4 includes the results of testing of this lubricant composition in an engine test (Sequence 3E test). The test (according to ASTM STP 315H PtII) was used to determine, among other aspects of lubricant performance, resistance to oxidation as measured by viscosity increase:

**TABLE 4**

| Composition | Compound contained in composition | %w compound in composition | Test number in run sequence | Viscosity increase at 64 hours, % |
|---|---|---|---|---|
| L* | None | 0 | 1 | 525 |
| L* | None | 0 | 4 | 4667 |
| M | Example 1 | 17 | 2 | 11 |
| M | Example 1 | 17 | 3 | 11 |
| API SH PASS LIMIT | | | | 375 max |

| | | | | |
|---|---|---|---|---|
| *: Comparative | | | | |

Results in this engine test for a lubricating composition containing the same concentrations of the additive Package A, pour point depressant and Viscosity Index improving polymer, but blended in the hydrocarbon base stock only (Composition L), are included for comparison.

The test results show that relative to the analogous lubricant composition without added compound of the present invention, the lubricant composition containing the compound of Example 1 gave a very large benefit in oxidation stability in the engine test. The lubricant composition without said compound gave failing results in both tests, while the lubricant composition containing said compound showed almost no change in viscosity during the test.

The compounds of the present invention have a polar nature imparted by, for example, a carboxylic ester function. The polarity of base fluids affects, among other aspects of lubricant performance, the behaviour of elastomer seals in engines and machines. The compound of Example 1, which contains 2 ester groups per molecule, was compared with the fluid of Comparative D (2-(2-ethoxyethoxy)-ethyl-9(10)-(carbobutoxymethylthio)stearate) which has been esterified with an ethoxylated alcohol, thus modifying the polarity of the molecule. Tests were carried out according to CEC-L-39-T-87 using nitrile- and fluoroelastomer rubbers in a high performance gasoline engine lubricant, in which the base fluids in question were incorporated at two levels, 5% and 17% by weight. The results are shown in Table 5:

**TABLE 5**

| Lubricant composition | | | | | Specns. |
|---|---|---|---|---|---|
| Fluid in compn. | Ex. 1 | Ex. 1 | Comparative D | Comparative D | Min/ Max |
| Conc.of base fluid in compn. %w | 5 | 17 | 5 | 17 | |

| FLUORO ELASTOMER RE-1 | | | | | |
|---|---|---|---|---|---|
| Tensile strength % | -0.7 | -4.4 | -9.3 | -18.3 | -50/0 |
| Elongation % | -3.7 | -5.5 | -22.7 | -21.2 | -60/0 |
| Volume change % | 2 | 1 | 1 | 1 | 0/5 |
| Hardness pts. | 2 | 1 | 0 | -2* | 0/5 |

| NITRILE ELASTOMER RE-4 | | | | | |
|---|---|---|---|---|---|
| Tensile strength % | -2.2 | -1.6 | 0.3* | -1.1 | -20/0 |
| Elongation % | -31.7 | -26.5 | -22.9 | -9.0 | -50/0 |
| Volume change % | 2 | 1 | 3 | 7* | -5/5 |
| Hardness pts. | 0 | -1 | -3 | -7* | -5/5 |

| | | | | | |
|---|---|---|---|---|---|
| * out of specification | | | | | |

While the compositions containing the compound of Example 1 passed all criteria for the fluoro and nitrile seals at both concentrations of the compound, the compositions containing Comparative D failed for hardness with the fluoro elastomer at 17% by weight of the compound, and failed with the nitrile elastomer for volume change and hardness at 17% by weight of the compound and, marginally, for tensile strength at 5% by weight of the compound.

## Claims

1. A lubricating oil composition comprising as antioxidant base fluid a compound of the formula 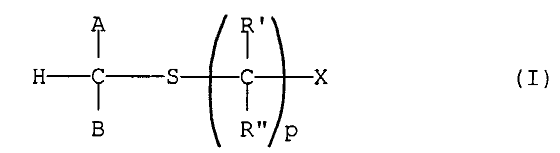 wherein
A is a hydrogen atom or an alkyl group;
B is an alkyl group; or a group of the formula CO₂-W, where W is an alkyl group; or a group of the formula Y-CO₂-Z, where Y is an alkylene group and Z is an alkyl group; or a group of the formula CH₂CH(CO₂D)CH₂CO₂E, where D and E are each independently alkyl groups;
R' and R" are each independently a hydrogen atom, an alkyl group or a group CH₂CO₂F, where F is an alkyl group;
p is 0 to 40;
X is a group CO₂G where G is a C₁₋₃₀ alkyl group, or a group (CH₂)ₙCO₂J where J is a C₁₋₁₀ alkyl group and n is 1 to 12;
said compound having a molecular weight of at least 440; with the exclusion of
(a) compounds of the formula S(CH₂CH₂CO₂R)₂ or S(CH₂CO₂R)₂ , wherein R is an alkyl group,
(b) compounds of the formula 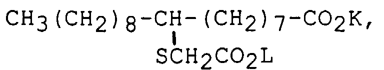 wherein K and L are each an octyl group,
(c) a compound of the formula 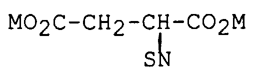 wherein M is a decyl group and N is a dodecyl group, and (d) alkyl beta-thio propionic acid ester selected from the group consisting of R₁SCH₂CH₂CO₂R₂ wherein R₁ and R₂ are alkyl groups containing from 1 to 30 carbon atoms, and
wherein the said compound is present in the amount of 5 to 25% by weight of the total base fluid.

2. A lubricating oil composition according to claim 1 wherein the balance of the total base fluid is a base oil of mineral or synthetic origin.

3. Use of a compound of the formula 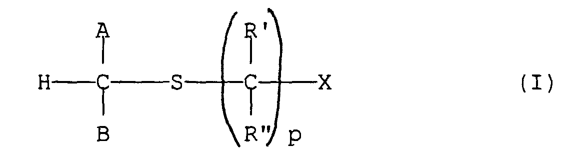 wherein
A is a hydrogen atom or an alkyl group;
B is an alkyl group; or a group of the formula CO₂-W, where W is an alkyl group; or a group of the formula Y-CO₂-Z, where Y is an alkylene group and Z is an alkyl group; or a group of the formula CH₂CH(CO₂D)CH₂CO₂E, where D and E are each independently alkyl groups;
R' and R" are each independently a hydrogen atom, an alkyl group or a group CH₂CO₂F, where F is an alkyl group;
p is 0 to 40;
X is a group CO₂G where G is a C₁₋₃₀ alkyl group, or a group (CH₂)ₙCO₂J where J is a C₁₋₁₀ alkyl group and n is 1 to 12;
with the exclusion of compounds of the formula S(CH₂CH₂CO₂R)₂ or S(CH₂CO₂R)₂, wherein R is an alkyl group;
as an antioxidant base fluid in a lubricating oil composition.

## Patentansprüche

1. Schmierölzusammensetzung, die als Antioxidationsgrundfluid eine Verbindung der Formel 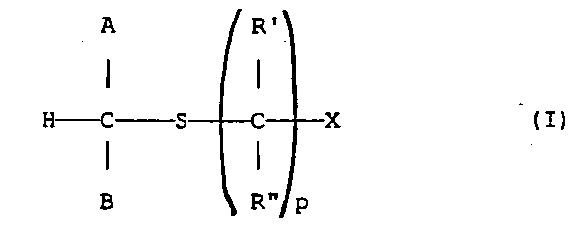 umfaßt, worin
A ein Wasserstoffatom oder eine Alkylgruppe ist;
B eine Alkylgruppe; oder eine Gruppe der Formel CO₂-W, worin W eine Alkylgruppe darstellt; oder eine Gruppe der Formel Y-CO₂-Z, worin Y eine Alkylengruppe ist und Z eine Alkylgruppe darstellt; oder eine Gruppe der Formel CH₂CH(CO₂D)CH₂CO₂E ist, worin D und E jeweils unabhängig Alkylgruppen bezeichnen;
R' und R" jeweils unabhängig ein Wasserstoffatom, eine Alkylgruppe oder eine Gruppe CH₂CO₂F bedeuten, worin F eine Alkylgruppe bezeichnet;
p für 0 bis 40 steht;
X eine Gruppe CO₂G darstellt, worin G eine C₁₋₃₀Alkylgruppe oder eine Gruppe (CH₂)ₙCO₂J bezeichnet, worin J eine C₁₋₁₀Alkylgruppe ist und n den Wert 1 bis 12 hat;
wobei diese Verbindung ein Molekulargewicht von wenigstens 440 aufweist;
ausgenommen
(a) Verbindungen der Formel S(CH₂CH₂CO₂R)₂ oder S(CH₂CO₂R)₂, worin R eine Alkylgruppe darstellt,
(b) Verbindungen der Formel 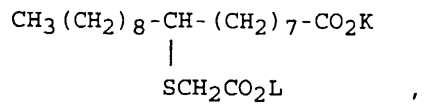 worin K und L jeweils eine Octylgruppe sind oder worin K eine Butylgruppe darstellt und L eine Ethylgruppe ist,
(c) eine Verbindung der Formel 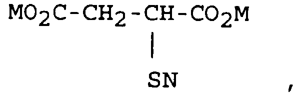 worin M eine Decylgruppe ist und N eine Dodecylgruppe ist, und
(d) Alkyl-beta-thiopropionsäureester, ausgewählt aus der aus R₁SCH₂CH₂CO₂R₂ bestehenden Gruppe, worin R₁ und R₂ Alkylgruppen mit 1 bis 30 Kohlenstoffatomen darstellen,
und worin diese Verbindung in einer Menge von 5 bis 25 Gew.-% des Gesamtgrundfluids zugegen ist.

2. Schmieölzusammensetzung nach Anspruch 1, worin der Rest des Gesamtgrundfuids ein Grundöl mineralischen oder synthetischen Ursprung ist.

3. Verwendung einer Verbindung mit der Formel 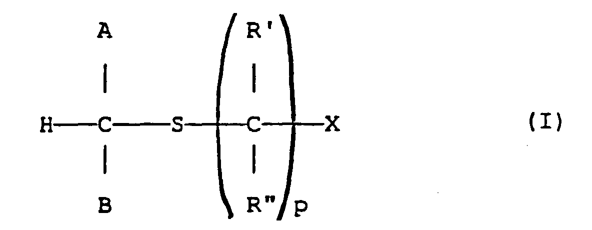 als ein antioxidierendes Grundfluid in einer Schmierölzusammensetzungen, wobei diese Verbindung in einer Menge von 5 bis 25 Gew.-% des Gesamtgrundfluids zugegen ist, in welcher Formel
A ein Wasserstoffatom oder eine Alkylgruppe ist;
B eine Alkylgruppe; oder eine Gruppe mit der Formel CO₂-W, worin W eine Alkylgruppe darstellt; oder eine Gruppe mit der Formel Y-CO₂-Z, worin Y eine Alkylengruppe ist und Z eine Alkylgruppe darstellt; oder eine Gruppe mit der Formel CH₂CH(CO₂D)CH₂CO₂E ist, worin D und E jeweils unabhängig Alkylgruppen darstellen;
R' und R" jeweils unabhängig ein Wasserstoffatom, eine Alkylgruppe oder eine Gruppe CH₂CO₂F bedeuten, worin F eine Alkylgruppe darstellt;
p den Wert 0 bis 40 hat;
X eine Gruppe CO₂G ist, worin G eine C₁₋₃₀Alkylgruppe darstellt, oder eine Gruppe (CH₂)ₙCO₂J ist, worin J eine C₁₋₁₀Alkylgruppe bezeichnet und n den Wert 1 bis 12 hat; ausgenommen Verbindungen mit der Formel S(CH₂CH₂CO₂R)₂ oder S(CH₂CO₂R)₂, worin R eine Alkylgruppe bezeichnet.

## Revendications

1. Composition d'huile lubrifiante comprenant comme fluide de base antioxydant un composé de la formule : 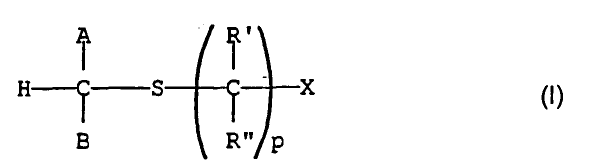 dans laquelle :
A est un atome d'hydrogène ou un groupe alkyle;
B est un groupe alkyle ou un groupe de la formule CO₂-W, où W est un groupe alkyle, ou un groupe de la formule Y-CO₂-Z, où Y est un groupe alkylène et Z est un groupe alkyle, ou un groupe de la formule CH₂CH(CO₂D)CH₂CO₂E, où D et E représentent chacun indépendamment des groupes alkyle;
R' et R" représentent chacun indépendamment un atome d'hydrogène, un groupe alkyle ou un groupe CH₂CO₂F, où F est un groupe alkyle;
p vaut de 0 à 40;
X est un groupe CO₂G où G est un groupe alkyle en C₁₋₃₀, ou un groupe (CH₂)ₙCO₂J où J est un groupe alkyle en C₁₋₁₀ et n vaut de 1 à 12;
ledit composé ayant un poids moléculaire d'au moins 440; à l'exclusion :
(a) des composés de la formule S(CH₂CH₂CO₂R)₂ ou S(CH₂CO₂R)₂, dans laquelle R est un groupe alkyle,
(b) des composés de la formule 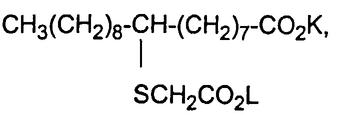 dans laquelle K et L sont chacun un groupe octyle,
(c) des composés de la formule 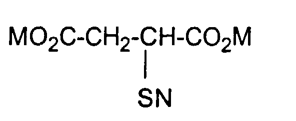 dans laquelle M est un groupe décyle et N est un groupe dodécyle, et
(d) des esters alkyliques d'acide bêta-thiopropionique choisis dans le groupe comprenant R₁SCH₂CH₂CO₂R₂, dans lequel R₁ et R₂ sont des groupes alkyle contenant de 1 à 30 atomes de carbone, et
dans laquelle le composé précité est présent à raison de 5 à 25% en poids par rapport au fluide de base total.

2. Composition d'huile lubrifiante suivant la revendication 1, dans laquelle le restant du fluide de base total est une huile de base d'origine minérale ou synthétique.

3. Utilisation d'un composé de la formule : 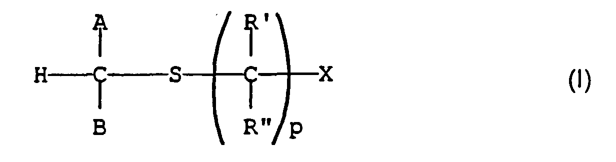 dans laquelle :
A est un atome d'hydrogène ou un groupe alkyle;
B est un groupe alkyle ou un groupe de la formule CO₂-W, où W est un groupe alkyle, ou un groupe de la formule Y-CO₂-Z, où Y est un groupe alkylène et Z est un groupe alkyle, ou un groupe de la formule CH₂CH(CO₂D)CH₂CO₂E, où D et E représentent chacun indépendamment des groupes alkyle;
R' et R" représentent chacun indépendamment un atome d'hydrogène, un groupe alkyle ou un groupe CH₂CO₂F, où F est un groupe alkyle;
p vaut de 0 à 40;
X est un groupe CO₂G où G est un groupe alkyle en C₁₋₃₀, ou un groupe (CH₂)ₙCO₂J où J est un groupe alkyle en C₁₋₁₀ et n vaut de 1 à 12;
à l'exclusion des composés de la formule S(CH₂CH₂CO₂R)₂ ou S(CH₂CO₂R)₂, dans laquelle R est un groupe alkyle;
comme fluide base antioxydant dans une composition d'huile lubrifiante.
